(19) **Europäisches Patentamt**
**European Patent Office**
**Office européen des brevets**

(11) **EP 2 158 840 B1**

(12) **EUROPÄISCHE PATENTSCHRIFT**

(45) Veröffentlichungstag und Bekanntmachung des Hinweises auf die Patenterteilung:
**10.09.2014 Patentblatt 2014/37**

(51) Int Cl.:
***A61B 5/00*** *(2006.01)*

(21) Anmeldenummer: **09166549.7**

(22) Anmeldetag: **28.07.2009**

(54) **Implantierbarer Biosensor und Sensoranordnung**

Implantable biosensor and sensor assembly

Biocapteur implantable et agencement de capteur

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **27.08.2008 DE 102008039858**

(43) Veröffentlichungstag der Anmeldung:
**03.03.2010 Patentblatt 2010/09**

(73) Patentinhaber: **Biotronik CRM Patent AG**
**6341 Baar (CH)**

(72) Erfinder:
* **Skerl, Olaf**
**18209 Bad Doberan (DE)**
* **Urbaszek, Albrecht**
**91336 Heroldsbach (DE)**
* **Czygan, Gerald**
**91054 Buckenhof (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
WO-A1-2009/025563    US-A1- 2004 045 879
US-A1- 2005 061 079    US-A1- 2006 258 761
US-A1- 2007 282 172    US-B1- 6 475 750

* **LANGE K ET AL: "Influence of intermediate aminodextran layers on the signal response of surface acoustic wave biosensors", ANALYTICAL BIOCHEMISTRY, ACADEMIC PRESS INC, NEW YORK, Bd. 377, Nr. 2, 15. Juni 2008 (2008-06-15) , Seiten 170-175, XP022701426, ISSN: 0003-2697, DOI: 10.1016/J.AB.2008.03.012 [gefunden am 2008-03-14]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung liegt auf dem Gebiet der Sensortechnik und betrifft einen implantierbaren, drahtlos auslesbaren Biosensor sowie eine mit zumindest einem solchen Biosensor ausgestattete Sensoranordnung.

Für die Detektion der Anwesenheit von Analyten und der Bestimmung ihrer Konzentrationen in Körperflüssigkeiten wie beispielsweise Blut existieren vielfältige Analysemethoden und die dafür notwendige Gerätetechnik. Allgemein werden dafür Flüssigkeitsproben entnommen, die gegebenenfalls speziell aufbereitet und dann der Analyse zugeführt werden. Die Bandbreite erstreckt sich dabei von einfachen Teststreifen, die beispielsweise durch eine Farbänderung das Vorhandensein und die Konzentration eines spezifischen Stoffes (beispielsweise Glukose) anzeigen bis hin zu aufwändigen Analysen in Speziallabors. Ein wesentlicher Nachteil dieser Methoden ist, dass nur zu bestimmten (diskreten) Zeitpunkten Proben entnommen werden, die dann mehr oder weniger aufwändig analysiert werden. Besonders bei Laboranalysen liegt zudem meist eine relativ große Zeitspanne zwischen der Probenentnahme und der Verfügbarkeit des Ergebnisses. Dadurch sind kontinuierliche Messungen mit diesen Methoden nicht möglich.

Oftmals ist eine kontinuierliche Messung wünschenswert, insbesondere wenn die gemessenen Werte zur Steuerung von Therapiegeräten benutzt werden sollen. Als Beispiel für eine Erkrankung, bei der eine kontinuierliche Messung vorteilhaft ist, sei Diabetes mellitus genannt. Diabetes mellitus ist eine weit verbreitete Erkrankung mit ca. 250 Mio. Fällen weltweit, was ca. 6% der Weltbevölkerung entspricht. Im Zusammenhang mit Diabetes mellitus treten häufig Komplikationen und Folgeerkrankungen auf. Ein Beispiel dafür sind die bei Diabetes mellitus gehäuft auftretenden Herzkrankheiten. Die Therapie von Diabetes mellitus erfolgt üblicherweise durch eine Insulingabe, für die eine Messung des Blutzuckerspiegels notwendig ist. Je häufiger der Blutzuckerspiegel gemessen wird, umso effizienter kann die Therapie erfolgen. Für eine automatische Insulingabe, zum Beispiel durch Insulinpumpen, ist eine kontinuierliche Messung des Blutzuckerspiegels erforderlich, um eine optimierte Insulindosierung entsprechend den Erfordernissen des Patienten zu ermöglichen.

Weiterhin ist eine kontinuierliche Überwachung der Konzentration von Enzymen, Hormonen, Markermolekülen, pH-Werten oder Glukosekonzentrationen wünschenswert, um krankhafte Veränderungen rechtzeitig zu erkennen und die erforderlichen Therapien einleiten zu können.

Aus diesem Grund haben Biosensoren eine große Bedeutung bei der Detektion von Enzymen, Hormonen, Markermolekülen, pH-Werten oder Glukosekonzentrationen erlangt. Chemische und biologische Sensoren basieren auf dem Grundprinzip, die Konzentration oder das Vorhandensein eines bestimmten Stoffes selektiv in die Änderung einer physikalischen Größe umzuwandeln, die dann beispielsweise elektrisch messbar ist. Bei diesen physikalischen Größen kann es sich beispielsweise um die Wellenlänge von absorbiertem oder emittiertem Licht, Druck, Viskosität, Masse aber auch um elektrische Größen wie Ströme, Spannungen oder Impedanzen handeln.

Eine Möglichkeit zur Realisierung von Sensoren sind Hydrogele. Hydrogele sind spezielle Polymere, die in der Lage sind, ein Lösungsmittel, beispielsweise Wasser, in einer Menge des Vielfachen ihres Eigenvolumens aufzunehmen. Diese Eigenschaft entsteht durch die Verknüpfung der Polymermoleküle zu einem dreidimensionalen Netzwerk, in das sich die Lösungsmittelmoleküle (z. B. Wasser) einlagern können. Hydrogele können beispielsweise so gestaltet sein, dass sie auf die Änderung der Konzentrationen bestimmter Ionen (beispielsweise pH-Wert) oder bestimmter Stoffe (z. B. Glukose oder Hormone) mit relativ großen Änderungen ihres Volumens reagieren. Auch können gezielt Verbindungen eingebettet werden, welche eine Affinität zu bestimmten Stoffen haben (beispielsweise Concanavalin A für Glukose), um auf diese Weise die Eigenschaften des Hydrogels in Abhängigkeit der Konzentration dieses Stoffes zu beeinflussen. Über die Volumenänderung hinaus können sich bei der Lösungsmitteleinlagerung beispielsweise auch die Viskosität, die Dichte, und/oder optische und elektrische Eigenschaften der Hydrogele ändern. Aufgrund dieses Verhaltens sind Hydrogele als Basis für Bio- und chemische Sensoren geeignet. Gemessen wird dabei die Änderung einer bestimmten Eigenschaft des Hydrogels (z. B. dessen Viskosität), um hieraus eine Konzentration des zu bestimmenden Stoffes zu ermitteln.

Dem Fachmann sind weiterhin so genannte SAW-Sensoren (SAW = Surface Acoustic Wave) bekannt. Allgemein bestehen SAW-Sensoren aus einem piezoelektrischen Substrat, auf dessen Oberfläche so genannte Interdigitalelektroden (IDT = Inter Digital Transducer) aufgebracht sind. Legt man an einen IDT ("Eingangs-IDT") eine Wechselspannung an, werden über den inversen Piezoeffekt akustische Oberflächenwellen erzeugt, die sich auf der Oberfläche des Substrats ausbreiten. Diese Oberflächenwellen können an einem oder mehreren akustischen Reflektoren (z. B. einem IDT) reflektiert werden und erzeugen dann in dem Eingangs-IDT über den Piezoeffekt eine Wechselspannung. Wird beispielsweise über eine Antenne ein Wechselspannungsimpuls in den Eingangs-IDT eingekoppelt, wird er in ein akustisches Wellenpaket ungewandelt, das nach Reflexion am akustischen Reflektor einen Wechselspannungsimpuls im Eingangs-IDT erzeugt, der über diese Antenne abgestrahlt wird, ähnlich einem Echo. Für das Zurücksenden dieses Echos wird keine zusätzliche Energie benötigt. SAW-Sensoren können als Verzögerungsleitung oder als so genannter One-Port-Resonator aufgebaut sein und können mit Frequenzen von ca. 30 MHz bis über 10 GHz betrieben werden.

Nun wird die Ausbreitung der akustischen Welle auf der Oberfläche des Substrats von verschiedenen Faktoren beeinflusst, wie beispielsweise der mechanischen Spannung im Substrat, der Temperatur, dem Massebelag auf der Oberfläche oder der akustischen Ankopplung an das umgebende Medium. Durch diese Einflüsse verändert sich unter anderem die Ausbreitungsgeschwindigkeit der akustischen Oberflächenwellen oder deren Dämpfung, wodurch sich die Eigenschaften des Echos, beispielsweise Laufzeit oder Amplitude, verändern.

Aufgrund dieser Eigenschaften ist es mittels SAW-Sensoren möglich, rein passive, drahtlos auslesbare Sensoren für unterschiedliche physikalische Größen zu realisieren, die mit einem hochfrequenten elektromagnetischen Impuls, vergleichbar einem Radar-Impuls, abgefragt werden können. Das empfangene Echo enthält die Information über den Messwert.

SAW-Sensoren können beispielsweise als Drucksensoren verwendet werden. Dabei wird das Substrat beispielsweise auf einer Membran angebracht, wodurch es geringfügig durchgebogen wird. Diese Verbiegung kann auch erreicht werden, indem die Kraft von der Membran über einen Stößel auf das geeignet gelagerte Substrat übertragen wird. Durch die Verbiegung des Substrats treten darin mechanische Spannungen auf, die beispielsweise die Ausbreitungsgeschwindigkeit der akustischen Welle beeinflussen. Dadurch ändert sich die Resonanzfrequenz des SAW-Sensors oder die Phasenlage des Echos relativ zum Anregungsimpuls in Abhängigkeit von der mechanischen Spannung, und folglich in Abhängigkeit von dem zu messenden Druck.

Zur Messung der Viskosität von Flüssigkeiten sind SAW-Sensoren ebenfalls gut geeignet. Hierbei finden vorrangig SAW-Anordnungen Verwendung, die auf der Basis akustischer Scherwellen (SH-Wellen) arbeiten. Scherwellen haben den Vorteil, dass sie akustisch nur über die Viskosität an die Flüssigkeit ankoppeln und somit die Dämpfung der Oberflächenwelle durch die angrenzende Flüssigkeit relativ gering ist. Die Eindringtiefe (d) der Oberflächenwelle in die Flüssigkeit ist von deren Viskosität ($\eta$), deren Dichte (p) und der Frequenz der Oberflächenwelle ($\omega$) abhängig:

$$d = \sqrt{\frac{2\eta}{\omega\rho}} \; .$$

Die Einkopplung der akustischen Welle in die Flüssigkeit führt damit effektiv zu einer Massebeladung der Oberfläche, durch die sich die Ausbreitungsgeschwindigkeit der akustischen Oberflächenwelle verändert, und auch zu einer Dämpfung der Welle. Diese Effekte können durch eine akustische Impedanz ($Z_a$) der Flüssigkeit beschrieben werden, die ebenfalls von der Viskosität ($\eta$), der Dichte (p) und der Frequenz ($\omega$) abhängig ist

$$Z_a = \sqrt{\omega\rho\eta} \; .$$

Dadurch ändert sich die Resonanzfrequenz des SAW-Sensors, die Phasenlage des Echos relativ zum Anregungsimpuls oder die Amplitude des Echos in Abhängigkeit von der Viskosität der angekoppelten Flüssigkeit.

Auch zur Messung von Leitfähigkeiten und Impedanzen sind SAW-Sensoren geeignet. Hierzu verbindet man die Anschlüsse eines der akustischen Reflektoren auf der Oberfläche des SAW-Sensors elektrisch mit der zu messenden Impedanz, beispielsweise definiert angeordnete Elektroden in einer Flüssigkeit. Dieser akustische Reflektor wird dadurch mit der elektrischen Impedanz (Z = R + jX) der Elektrodenanordnung belastet. Dadurch ändern sich die Amplitude und die Phasenlage des Echos dieses Reflektors abhängig von Betrag und Phase der elektrischen Impedanz der Elektrodenanordnung, die sowohl von der elektrischen Leitfähigkeit der Flüssigkeit als auch von deren dielektrischen Eigenschaften beeinflusst wird. Auch das Ausschwingverhalten des SAW-Sensors wird durch die Lastimpedanz beeinflusst, beispielsweise dessen Ausschwing-Zeitkonstante. Dadurch können die elektrischen Eigenschaften der Flüssigkeit, wie beispielsweise deren Leitfähigkeit oder Dielektrizitätskonstante, durch die Bestimmung der Parameter der empfangenen Echos gemessen werden.

SAW-Sensoren nach den beschriebenen Prinzipien haben sich bei industriellen Anwendungen bereits bewährt, beispielsweise als Reifendrucksensoren oder Ölqualitätssensoren.

Als kontinuierlich messende Verfahren sind Anwendungen auf Basis von SAW-Sensoren bekannt, bei denen die Masseanlagerung spezifischer Stoffe gemessen wird. Hierbei wird die Oberfläche des SAW-Sensors mit einem selektiven Material beschichtet, an das sich der Analyt anlagert. Der Massenzuwachs auf der Oberfläche des SAW-Sensors wird mit allgemein bekannten Methoden gemessen und daraus das Vorhandensein und die Konzentration des Analyten bestimmt. Ein häufiger Nachteil dieser Methode ist, dass die selektive Beschichtung altert, kontaminiert wird oder die Aufnahme des Analyten nicht reversibel ist, so dass die Messung nach einer bestimmten Nutzungsdauer nicht mehr mit ausreichender Genauigkeit möglich ist und die SAW-Sensoren ausgetauscht werden müssen.

Zur Überwindung des Nachteils einer geringen Nutzungsdauer sind Anordnungen bekannt, die auf der Basis von Hydrogelen arbeiten. So verändert sich beispielsweise die Viskosität eines Hydrogels beim Eindringen eines Analyten (beispielsweise Glukose) in die Polymermatrix. Vorteilhaft ist, dass dieser Prozess reversibel ist. Bei geeigneter Anordnung bildet sich ein veränderliches Konzentrationsgleichgewicht zwischen der Testflüssigkeit (Hydrogel) und der Umgebung aus. Über die Messung der Viskositätsänderung kann die Konzentration des Analyten in der Probeflüssigkeit bestimmt werden (Affinitätsviskosimetrie). Die Viskosität kann beispielsweise durch den Strömungswiderstand über einer durchströmten Kapillare gemessen werden. Nachteilig hierbei ist, dass eine Strömung in der sensitiven Flüssigkeit (bzw. Hydrogel) angetrieben werden muss und dass bei einigen Anordnungen die sensitive Flüssigkeit verbraucht wird. Diese Verfahren sind vorrangig für Labormessungen geeignet. Diesen Nachteil weisen Anordnungen, bei denen die Viskosität über das Schwingverhalten eines vom Hydrogel umschlossenen Biegebalkens bestimmt wird, nicht auf. Allerdings wird für die Anregung der Schwingungen des Biegebalkens, deren Messung und die Übertragung der Messwerte über eine Telemetrieverbindung relativ viel Energie benötigt, so dass die Lebensdauer und die Baugröße eines implantierbaren Sensors nach diesem Prinzip durch dessen Batteriekapazität bestimmt werden. Andernfalls muss von Extern Energie für den Betrieb des Sensors übertragen werden, wobei der Sensor dann Mittel zum Empfangen und Speichern dieser Energie enthalten muss.

Weiterhin sind Anordnungen bekannt, die die Volumenänderungen von Hydrogelen durch die Einlagerung eines Analyten zur Konzentrationsbestimmung nutzen. Dabei befindet sich das Hydrogel in einer mit einer festen semipermeablen Membran abgeschlossenen Kapsel. Durch das Quellen des Hydrogels verändert sich der Druck in dieser Kapsel, der mittels eines beliebigen Druckaufnehmers gemessen wird. Ebenso kann der durch den Konzentrationsunterschied innerhalb und außerhalb der Kapsel entstehende osmotische Druck für die Konzentrationsbestimmung des Analyten verwendet werden. Die Signale des Druckaufnehmers werden im Sensor von einer Messelektronik verarbeitet und über eine Telemetrieverbindung aktiv übertragen. Die hierfür benötigte Energie muss wiederum durch eine Batterie oder durch eine Energieübertragung bereitgestellt werden. Auch andere bekannte implantierbare Sensoren zur Konzentrationsbestimmung eines Analyten, beispielsweise in Form eines Stents, weisen den Nachteil auf, dass sie für den Betrieb der Messelektronik und der aktiven Telemetrie eine Batterie oder eine Möglichkeit zur Energieübertragung benötigen.

US 6,475,750 B1 zeigt einen implantierbaren Biosensor, welcher eine mit einem Hydrogel gefüllte Messkammer umfasst, wobei das Hydrogel in der Lage ist, eine Änderung der Konzentration eines vorbestimmten Analyten oder Ionensorte in eine Druckänderung umzuwandeln, und wobei die Messkammer durch wenigstens eine Membran abgeschlossen ist, die für den Analyten oder Ionensorte durchlässig und für das Hydrogel undurchlässig ist, wobei der Biosensor mit wenigstens einem mikroakustischen Sensor ausgerüstet ist, der mit dem Hydrogel so wirkverbunden ist, dass er die mit der Konzentration des Analyten oder Ionensorte sich ändernde Druckänderung detektieren kann.

Wünschenswert ist es, einen implantierbaren, rein passivern, mittels elektromagnetischen Impulsen drahtlos auslesbaren Sensor zur Bestimmung der Konzentration eines Analyten, beispielsweise Glukose, in Körperflüssigkeiten zu realisieren. Die Baugröße und die Lebensdauer des Sensors werden dabei nicht durch die Größe der Batterie beziehungsweise des Energiespeichers und der Messelektronik beschränkt.

Diese und weitere Aufgaben werden nach dem Vorschlag der Erfindung durch einen implantierbaren Biosensor gemäß Anspruch 1 und eine Sensoranordnung gemäß Anspruch 5 gelöst. Vorteilhafte Ausgestaltungen der Erfindung sind durch die Merkmale der Unteransprüche angegeben.

Gemäß der Erfindung ist ein implantierbarer, drahtloser bzw. drahtlos auslesbarer Biosensor zur medizinischen Anwendung gezeigt. Der Biosensor umfasst eine mit einer Testflüssigkeit gefüllte Messkammer. Die Testflüssigkeit ist in der Lage, eine Änderung der Konzentration eines vorbestimmten Analyten oder Ionensorte in eine Änderung einer physikalischen Größe umzuwandeln. Hierbei handelt es sich vorzugsweise um ein Hydrogel. Die Messkammer ist durch wenigstens eine so genannte semipermeable Membran abgeschlossen, das heißt, die Membran ist für den vorbestimmten Analyten oder Ionensorte durchlässig und für die Testflüssigkeit undurchlässig. Zudem ist der Biosensor mit wenigstens einem mikroakustischen Sensor, beispielsweise einem SAW-Sensor, ausgerüstet, der mit der Testflüssigkeit so wirkverbunden bzw. gekoppelt ist, dass er die mit der Konzentration des vorbestimmten Analyten oder Ionensorte sich ändernde physikalische Größe der Testflüssigkeit detektieren kann.

Hier und im Weiteren soll unter einem "mikroakustischen Sensor" ein Sensor verstanden sein, der einen elektromagnetischen Impuls in eine akustische Welle bzw. eine akustische Welle in einen elektromagnetischen Impuls umwandeln kann. Hierbei kann es sich insbesondere um einen eingangs erläuterten SAW-Sensor handeln.

Der implantierbare Biosensor weist eine mit der Testflüssigkeit gefüllte erste Kammer und eine mit der Testflüssigkeit nicht gefüllte zweite Kammer auf, wobei die beiden Kammern wenigstens abschnittsweise durch eine bei einem Druckunterschied in den beiden Kammern sich verformende erste Druckmessmembran voneinander getrennt sind. Zudem ist die zweite Kammer wenigstens abschnittsweise durch eine bei einem Druckunterschied der zweiten Kammer gegenüber der Umgebung sich verformende zweite Druckmessmembran von der Umgebung getrennt. Hierbei ist ein mikroakustischer Sensor, beispielsweise SAW-Sensor, in der zweiten Kammer aufgenommen und so mit den beiden Druckmessmembranen wirkverbunden bzw. gekoppelt, dass er deren Verformung detektieren kann.

Bei einer Ausgestaltung des erfindungsgemäßen Biosensors kann es vorteilhaft sein, wenn der mikroakustische Sensor als ein mit der wirkverbundenen Druckmessmembran mechanisch gekoppeltes biegbares Organ, beispielsweise in Form eines Biegebalkens, ausgebildet ist.

Bei einer Ausgestaltung des erfindungsgemäßen Biosensors kann es weiterhin vorteilhaft sein, wenn ein (gegebenenfalls weiterer) mikroakustischer Sensor in der zweiten Kammer aufgenommen ist, der die Temperatur detektieren kann.

Bei einer Ausgestaltung des erfindungsgemäßen Biosensors kann es darüber hinaus vorteilhaft sein, wenn die Testflüssigkeit so ausgebildet ist, dass sie eine Änderung der Konzentration eines Analysten, insbesondere Glukose, oder Ionensorte in eine Änderung ihres Volumens umwandeln kann.

Gemäß einem zweiten Aspekt der Erfindung ist eine Sensoranordnung zur medizinischen Anwendung gezeigt. Die Sensoranordnung umfasst wenigstens einen wie oben beschriebenen implantierbaren Biosensor sowie eine mit dem Biosensor drahtlos gekoppelte Auslese- bzw. Abfrageeinrichtung, in der die vom Biosensor emittierten Signale empfangen und hinsichtlich einer Bestimmung der Konzentration des vorbestimmten Analyten oder Ionensorte analysiert werden. Hierbei kann vorzuziehen sein, wenn die Abfrageeinrichtung in ein Therapie- bzw. Patientengerät integriert ist. Unter einem "Therapiegerät" wird hier und im Weiteren ein Gerät verstanden, durch das ein therapeutischer Nutzen erzielbar ist, beispielsweise indem eine therapeutische Maßnahme vorgeschlagen oder durchgeführt wird, wie etwa die selbsttätige Abgabe von Insulin bei Diabetes mellitus. Zu diesem Zweck kann das Therapiegerät im Körper implantiert sein. Denkbar ist jedoch auch, dass das Therapiegerät nicht implantiert ist. Hierbei kann es weiterhin vorzuziehen sein, wenn das Therapie- bzw. Patientengerät mit einem Heimüberwachungssystem (Home-Monitoring-System) zur Überwachung der Konzentrationen vorbestimmter Analyten oder Ionensorten eines Patienten gekoppelt ist.

Die Erfindung wird nun anhand von Ausführungsbeispielen näher erläutert, wobei Bezug auf die beigefügten Zeichnungen genommen wird. Es zeigen:

Fig. 1    eine schematische perspektivische Ansicht eines Ausführungsbeispiels der erfindungsgemäßen Sensoranordnung;

Fig. 2    eine schematische Schnittdarstellung eines Biosensors;

Fig. 3    eine schematische Schnittdarstellung eines zweiten Biosensors;

Fig. 4    eine schematische Schnittdarstellung eines dritten Biosensors.

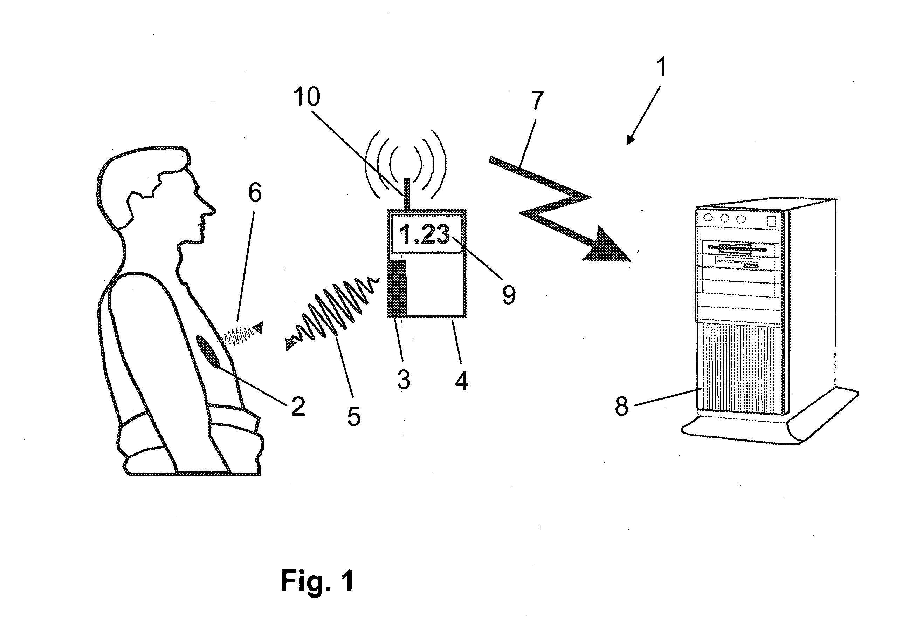

Sei zunächst Figur 1 betrachtet, worin in einer schematischen perspektivischen Ansicht ein Ausführungsbeispiel der erfindungsgemäßen Sensoranordnung veranschaulicht ist.

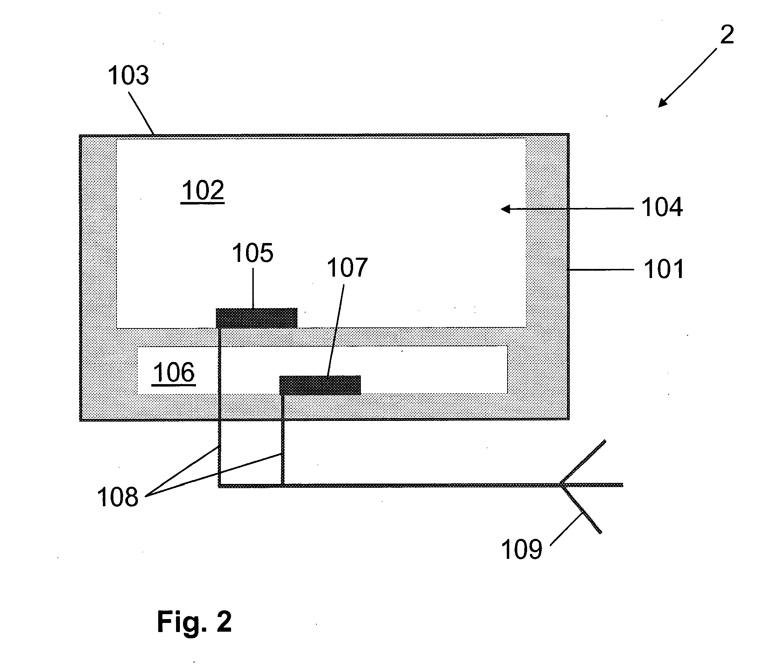

Demnach umfasst die insgesamt mit der Bezugszahl 1 bezeichnete Sensoranordnung einen im Körper eines Patienten implantierten Biosensor 2, ein mit dem Biosensor 2 drahtlos koppelbares Gerät, im Weiteren als "Patientengerät" 4 bezeichnet, das eine zum Auslesen des Biosensors 2 dienende Abfrageeinheit 3 enthält, sowie ein mit dem Patientengerät 4 beispielsweise drahtlos koppelbares Heimüberwachungssystem bzw. Home-Monitoring-System 8. Der Biosensor 2 umfasst eine in einer Messkammer aufgenommene Testflüssigkeit, beispielsweise ein Hydrogel, die auf eine Änderung der Konzentration eines vorbestimmten Analyten oder einer Ionensorte mit einer physikalischen Zustandsänderung antworten kann und ist zur Detektion dieser Zustandsänderung mit wenigstens einem mikroakustischen Sensor, beispielsweise einem SAW-Sensor, versehen, wie anhand der in den Figuren 2 bis 4 dargestellten Beispiele für den Biosensor 2 näher erläutert ist.

Der implantierte Biosensor 2 kann durch die im Patientengerät 4 enthaltene Abfrageeinheit 3 drahtlos abgefragt werden. Wie in Figur 1 beispielhaft dargestellt ist, kann sich die Abfrageeinheit 3 außerhalb des Körpers des Patienten im Patientengerät 4 befinden. Alternativ ist es jedoch gleichermaßen möglich, dass sich die Abfrageeinheit 3 in einem externen Therapiegerät (beispielsweise eine Insulinpumpe) oder innerhalb des Körpers in einem weiteren implantierten Gerät (beispielsweise ein Herzschrittmacher oder ein implantiertes Medikamentendepot) befindet. Ebenso kann der Biosensor 2 Bestandteil eines implantierbaren Gerätes sein, wobei dann die Abfrage der mikroakustischen Sensoren bzw. SAW-Sensoren auch drahtgebunden erfolgen kann.

Die Abfrageeinheit 3 enthält geeignete Mittel zum Senden eines hochfrequenten elektromagnetischen Impulses, im Weiteren als Sendeimpuls 5 bezeichnet, zum Empfangen des vom Biosensor 2 zurückgestrahlten Echos 6, und zur Signalverarbeitung und Messwertberechnung. Wie in Figur 1 gezeigt ist, ist das Patientengerät 4 mit einer Geräteantenne 10 zum Senden und Empfangen der elektromagnetischen Impulse 5, 6 ausgerüstet.

Eine drahtlose Abfrage des Biosensors 2 erfolgt mit dem Sendeimpuls 5, der von den SAW-Sensoren im Biosensor 2 modifiziert und als elektromagnetisches Echo 6 zurückgesendet wird ("SAW-Echo"). Die Abfrageeinheit 3 empfängt das SAW-Echo und bestimmt mit allgemein bekannten Methoden, auf die hier nicht näher eingegangen werden muss, geeignete Parameter der Echos und hieraus die Konzentrationen bestimmter Analyten bzw. Ionen. Als Echo-Parameter, die die Information über die zu messenden Konzentrationen der Analyten bzw. Ionen enthalten, können beispielsweise die Frequenzverschiebung, die Phasenverschiebung, Amplitudenverhältnisse, Laufzeiten oder Ausschwingzeitkonstanten verwendet werden.

Die Informationen über die Konzentrationen der Analyten bzw. Ionen können für die Steuerung einer entsprechenden Therapie oder zur Überwachung des Gesundheitszustands des Patienten verwendet werden. Die Therapiesteuerung kann in einem externen Therapiegerät oder direkt in einem implantierten Therapiegerät (IMD) erfolgen oder sie erfolgt über das externe Patientengerät 4, das die Abfrageeinheit 3 und eine (nicht dargestellte) Einheit für die Therapiesteuerung enthält und die Therapieinformationen über eine Kommunikationsverbindung an ein implantiertes Therapiegerät überträgt.

In dem in Figur 1 gezeigten Ausführungsbeispiel ist das Patientengerät 4 über eine beispielsweise drahtlose Kommunikationsverbindung 7 mit dem Home-Monitoring-System 8 verbunden. Denkbar wäre jedoch auch, dass das Patientengerät 4 über eine drahtgebundene Kommunikationsverbindung mit dem Home-Monitoring-System 8 datentechnisch verbunden ist.

Das Patientengerät 4 kann über eine Ein-Ausgabe-Einheit verfügen, wie beispielsweise ein Display 9, eine Folientastatur und einen akustischen Signalgeber. Die Informationen über die Konzentrationen von Analyten bzw. Ionen können vom Patientengerät 4 durch das Display 9 angezeigt werden. Zudem können diese Informationen über die Kommunikationsverbindung 7 an das Home-Monitoring-System 8 übertragen werden.

Die Konzentrationsinformationen können im Patientengerät 4 und/oder im Home-Monitoring-System 8 mit geeigneten Schwellwerten verglichen werden und beispielsweise können hieraus Alarme oder Therapieanweisungen generiert werden. Der Vergleich mit den Schwellwerten und die Generierung der Alarme oder der Therapieanweisungen kann ganz oder teilweise im Patientengerät 4 oder im Home-Monitoring-System 8, mit oder ohne Interaktion eines Arztes, erfolgen.

Weiterhin können die Konzentrationsinformationen im Patientengerät 4 über einen geeigneten Zeitraum gespeichert und zu geeigneten Zeitpunkten an das Home-Monitoring-System 8 übertragen werden. Ebenso können aus den Konzentrationsinformationen geeignete Parameter oder Trends und aus den Trends wiederum Trendparameter bestimmt werden. Die Bestimmung der Parameter, Trends und Trendparameter kann ganz oder teilweise im Patientengerät 4 oder im Home-Monitoring-System 8 erfolgen. Die Parameter oder die Trendparameter können ebenfalls auf geeignete Weise angezeigt oder zur Therapiesteuerung verwendet werden oder mit Schwellwerten verglichen werden, um daraus Alarme oder Therapiehinweise zu generieren. Dies kann wiederum ganz oder teilweise im Patientengerät 4 oder im Home-Monitoring-System 8 erfolgen.

Es wird nun Bezug auf Figur 2 genommen, worin anhand einer schematischen Schnittdarstellung ein Biosensor 2 veranschaulicht ist.

Der Biosensor 2 ist ein implantierbarer, drahtlos auslesbarer, passiver Sensor, der vorrangig zur Bestimmung des Glukosegehalts im Blut oder in der Bindegewebsflüssigkeit eingesetzt werden kann. Der Biosensor 2 wird an geeigneter Stelle, beispielsweise unter der Haut, implantiert. Er umfasst eine Kapsel 101, die aus einem geeigneten biokompatiblen Material gefertigt ist und eine Messkammer bzw. erste Kammer 102 umgibt. Auf einer Seite ist die Kapsel 101 bzw. erste Kammer 102 von einer geeigneten halbdurchlässigen Membran 103 abgeschlossen. Die erste Kammer 102 der Kapsel 101 ist mit einer geeigneten Testflüssigkeit 104 bzw. Hydrogel gefüllt, beispielsweise mit einer Lösung aus Dextran und Concanavalin A, wobei die Testflüssigkeit 104 die Eigenschaft besitzt, ihre Viskosität in Abhängigkeit der Glukosekonzentration zu verändern. Die Membran 103 ist so beschaffen, dass sie den Durchtritt von Glukosemolekülen ermöglicht und gleichzeitig den Austritt der Testflüssigkeit 104 verhindert, so dass sich ein Gleichgewicht der Glukosekonzentration zwischen der Testflüssigkeit 104 in der ersten Kammer 102 und der umgebenden Körperflüssigkeit ausbildet. Eine Änderung der Glukosekonzentration in der Testflüssigkeit 104 führt zu einer Veränderung von dessen Viskosität. Somit kann über eine Bestimmung der Viskosität der Testflüssigkeit 104 die Glukosekonzentration in der Körperflüssigkeit ermittelt werden.

Die Bestimmung der Viskosität der Testflüssigkeit 104 erfolgt mit einem ersten SAW-Sensor 105, der an einer geeigneten Stelle in der ersten Kammer 102 der Kapsel 101 derart angebracht ist, dass seine sensitive Oberfläche mit der Testflüssigkeit 104 in Kontakt ist, wobei die Ausbreitung der akustischen Wellen auf der sensitiven Oberfläche durch die Viskosität der Testflüssigkeit 104 beeinflusst wird.

Ein zweiter SAW-Sensor 107 ist so angebracht, dass seine sensitive Oberfläche keinen Kontakt zur Testflüssigkeit 104 hat. Wie in Figur 2 beispielhaft gezeigt, ist der zweite SAW-Sensor 107 zu diesem Zweck in einer zusätzlichen, abgeschlossenen, vorzugsweise evakuierten zweiten Kammer 106 aufgenommen. Die Wellenausbreitung auf der sensitiven Oberfläche des zweiten SAW-Sensors 107 wird dadurch nur von der Temperatur beeinflusst und dient zur passiven drahtlosen Temperaturmessung für die Temperaturkorrektur der Viskositätsmessung des ersten SAW-Sensors 105.

Beide SAW-Sensoren 105, 107 sind mit entsprechenden Kabeln 108 mit einer oder mehreren geeigneten Sensorantennen 109 verbunden. Über diese Sensorantenne(n) 109 wird der Sendeimpuls 5 der Abfrageeinheit 3 eingekoppelt und das Echo 6 der beiden SAW-Sensoren 105, 107 wieder abgestrahlt, um von der Auswerte- bzw. Abfrageeinheit 3 empfangen und verarbeitet zu werden. Durch eine geeignete Gestaltung der beiden SAW-Sensoren 105, 107, beispielsweise durch unterschiedliche Resonanzfrequenzen, wird eine Zuordnung der empfangenen Echos 6 zu den entsprechenden SAW-Sensoren 105, 107 ermöglicht.

Die Auswerte- bzw. Abfrageeinheit 3, die beispielsweise Bestandteil des externen Therapie- bzw. Patientengeräts 4, wie etwa einer Insulinpumpe, ist, sendet einen oder mehrere Sendeimpulse 5 aus und empfängt das bzw. die Echos 6 vom implantierten Biosensor 2. Mindestens ein Echo 6 enthält die Information über die Viskosität der Testflüssigkeit 104 und mindestens ein Echo 6 enthält die Temperaturinformation. Die Abfrageeinheit 3 ermittelt diese Informationen aus den Eigenschaften der Echos 6, beispielsweise über eine Bestimmung der Verschiebung der Echofrequenzen, erzeugt jeweils einen Messwert für die Viskosität und einen für die Temperatur und führt eine Temperaturkorrektur des Viskositätsmesswertes durch. Aus dem temperaturkorrigierten Viskositätsmesswert wird dann mittels des Zusammenhangs zwischen Viskosität und Glukosekonzentration die Glukosekonzentration in der Körperflüssigkeit ermittelt und zur weiteren Verwendung an das Therapie- bzw. Patientengerät 4 übermittelt. Diese Abfrage kann zyklisch, beispielsweise alle 5 Minuten, in bestimmten Intervallen oder zu bestimmten Zeitabschnitten (beispielsweise am Morgen, zu Mittag, am Abend und Mitternacht) erfolgen. Das Therapie- bzw. Patientengerät 4 bestimmt aus den Werten für die Glukosekonzentration die notwendigen Therapieparameter, beispielsweise die Dosierung der Insulingabe. Die gemessene Glukosekonzentration kann auf dem Display 9 des Therapie- bzw. Patientengerätes 4 angezeigt werden, im Patientengerät 4 abgespeichert und über die Kommunikationsverbindung 7 an das Home-Monitoring-System 8 übertragen werden. Aus den Glukosekonzentrationen können Trends und Trendparameter im Patientengerät 4 oder im Home-Monitoring-System 8 ermittelt werden, die wiederum auf dem Display 9 des Patientengeräts 4 angezeigt werden können. Auch können die Glukosekonzentrationen oder die Trendparameter im Patientengerät 4 oder im Home-Monitoring-System 8 mit geeigneten Schwellwerten verglichen und Alarmmeldungen generiert werden. Die Glukosewerte und Trends können auch zum Monitoring des Krankheitsverlaufs oder der Therapie durch den Patienten oder den behandelnden Arzt verwendet werden, beispielsweise die Einhaltung einer bestimmten Diät.

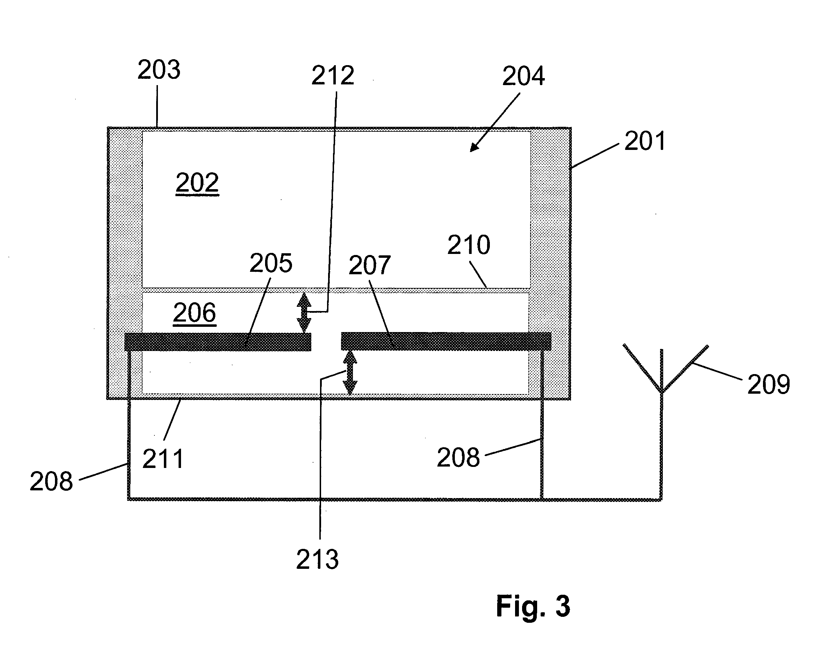

Es wird nun Bezug auf Figur 3 genommen, worin anhand einer schematischen Schnittdarstellung ein zweiter Biosensor 2 der veranschaulicht ist.

Der Biosensor 2 ist ein implantierbarer, drahtlos auslesbarer, passiver Sensor, der vorrangig zur Bestimmung des Glukosegehalts im Blut oder in der Bindegewebsflüssigkeit eingesetzt werden kann. Der Biosensor 2 wird an geeigneter Stelle, beispielsweise im Gewebe, implantiert. Er umfasst eine Kapsel 201, die aus einem geeigneten biokompatiblen Material gefertigt ist und eine Messkammer bzw. erste Kammer 202 umgibt. Auf einer Seite ist die Kapsel 201 bzw. erste Kammer 202 von einer geeigneten halbdurchlässigen, steifen Membran 203 abgeschlossen. Die erste Kammer 202 der Kapsel 201 ist mit einer geeigneten Testflüssigkeit 204 gefüllt, beispielsweise mit einer Mischung aus einem geeigneten Polymer und Concanavalin A als glukosebindendes Molekül, wobei die Testflüssigkeit 204 die Eigenschaft besitzt, ihr Volumen in Abhängigkeit der Glukosekonzentration zu verändern, d.h. zu quellen oder zu schrumpfen. Die Membran 203 ist so beschaffen, dass sie den Durchtritt von Glukosemolekülen ermöglicht und gleichzeitig den Austritt der Testflüssigkeit 204 verhindert, so dass sich ein Gleichgewicht der Glukosekonzentration zwischen der Testflüssigkeit 204 in der Kapsel 201 und der umgebenden Körperflüssigkeit ausbildet. In Abhängigkeit von der Glukosekonzentration quillt oder schrumpft die Testflüssigkeit 204, was zu Druckänderungen in der ersten Kammer 202 führt. Diese Druckänderungen können bei Verwendung einer geeigneten Testflüssigkeit 204 auch durch osmotischen Druck hervorgerufen werden, der ebenfalls von der Glukosekonzentration abhängig ist. Damit kann über die Messung der Druckdifferenz zwischen der erste Kammer 202 und der Umgebung der Glukosegehalt in der Körperflüssigkeit bestimmt werden.

Eine Messung der Druckdifferenz erfolgt mit mikroakustischen Sensoren, beispielsweise SAW-Sensoren 205, 207. Ein erster SAW-Sensor 205 dient der Messung des Druckes in der erste Kammer 202 und ein zweiter SAW-Sensor 207 dient der Messung des Umgebungsdruckes außerhalb des Biosensors 2. Ein weiterer SAW-Sensor (nicht dargestellt) kann in geeigneter Weise angeordnet werden, um zusätzlich die Temperatur der Anordnung zu bestimmen, so dass durch die erfasste Temperatur die Temperaturabhängigkeit der beiden SAW-Sensoren 205, 207 und die thermische Ausdehnung in der Messanordnung bei Bedarf kompensiert werden kann. Die beiden SAW-Sensoren 205, 207 sind mit entsprechenden Kabeln 208 mit einer oder mehreren geeigneten Sensorantennen 209 verbunden. Über die Sensorantenne(n) 209 wird der Sendeimpuls 5 der Abfrageeinheit 3 eingekoppelt und das Echo 6 der beiden SAW-Sensoren 205, 207 wieder abgestrahlt, um von der Auswerte- bzw. Abfrageeinheit 3 empfangen zu werden. Durch eine geeignete Gestaltung der beiden SAW-Sensoren 205, 207, beispielsweise durch unterschiedliche Resonanzfrequenzen, wird eine Zuordnung der empfangenen Echos 6 zu den entsprechenden SAW-Sensoren 205, 207 ermöglicht.

Die mit der Testflüssigkeit 204 gefüllte erste Kammer 202 wird zu diesem Zweck beispielsweise auf einer Seite von einer ersten, hermetisch dichten Druckmessmembran 210, auf die der Druck in der ersten Kammer 202 wirkt, abgeschlossen. Hinter ihr befindet sich eine zweite, beispielsweise evakuierte Kammer 206. In dieser zweiten Kammer 206 sind die beiden SAW-Sensoren 205, 207 für die Druckmessung und gegebenenfalls ein weiterer für die Temperaturmessung angeordnet. Diese zweite Kammer 206 wird nach außen hin durch eine zweite, hermetisch dichte Druckmessmembran 211 abgeschlossen, auf die der Umgebungsdruck wirkt.

Die beiden SAW-Sensoren 205, 207 sind beispielsweise als Biegebalken ausgeführt, dessen eines Ende in der Wand der Kapsel 201 fixiert und das andere Ende frei schwebend ist. Auf die frei schwebenden Enden der beiden SAW-Sensoren 205, 207 wird über geeignete erste bzw. zweite Stößel 212, 213 eine druckabhängige Verformung der zugehörigen Druckmessmembranen 210, 211 übertragen, wodurch die SAW-Sensoren 205, 207 geringfügig durchgebogen werden. Diese geringfügige Durchbiegung führt zu mechanischen Spannungen in den SAW-Sensoren 205, 207, wodurch die Ausbreitungseigenschaften der akustischen Wellen auf ihrer Oberfläche, beispielsweise deren Ausbreitungsgeschwindigkeit, beeinflusst werden.

Die spezielle Anordnung von Fig. 3 dient zur Illustration. Erfindungsgemäß wird eine Anordnung verwendet, mit der die Druckdifferenz zwischen der ersten Kammer 202 und der Umgebung gemessen werden kann. So kann beispielsweise der zweite Stößel 213, der den Umgebungsdruck überträgt, ebenfalls an das Ende des ersten SAW-Sensors 205 geführt werden, wodurch die Durchbiegung dieses SAW-Sensors 205 direkt durch die Druckdifferenz hervorgerufen wird. Der zweite SAW-Sensor 207 kann dann zur Temperaturmessung verwendet werden. Die Temperaturmessung kann beispielsweise auch ermöglicht werden, indem auf den beiden SAW-Sensoren 205, 207 mehrere Reflektoren an geeigneten Positionen angebracht werden.

Prinzipiell können für die Messung des Druckes in der ersten Kammer 202 und des Umgebungsdruckes auch andere Druckaufnehmer, beispielsweise kapazitive oder piezoresistive Drucksensoren, verwendet werden, bei denen Druckänderungen zur Änderung ihrer Impedanz führen. Diese Druckaufnehmer werden mit den Anschlüssen geeigneter Reflektoren auf der Oberfläche der beiden SAW-Sensoren 205, 207 verbunden, so dass deren Impedanzänderungen zur Änderung der Reflexionseigenschaften der Reflektoren führen.

Die Auswerte- bzw. Abfrageeinheit 3, die hier beispielsweise Bestandteil eines implantierten Therapiegeräts (z. B. ein implantiertes Insulindepot) ist, sendet einen oder mehrere Sendeimpulse 5 aus und empfängt das bzw. die Echos 6 vom implantierten Biosensor 2. Mindestens ein Echo 6 enthält die Information über den Druck in der ersten Kammer 202 und mindestens ein Echo enthält die Information über den Umgebungsdruck. Die Abfrageeinheit 3 ermittelt diese Informationen aus den Eigenschaften der Echos 6, beispielsweise über eine Bestimmung der Laufzeiten der Echos 6, erzeugt jeweils einen Messwert für den Druck in der ersten Kammer 202 und einen für den Umgebungsdruck. Aus der Differenz der beiden Druckmesswerte, die einer Temperaturkorrektur unterzogen werden können, wird auf geeignete Weise der Glukosegehalt in der Körperflüssigkeit bestimmt. Die Abfrage des Biosensors 2 kann zyklisch oder zu bestimmten Zeitpunkten erfolgen. Anhand der Glukosemesswerte steuert das implantierte Insulindepot beispielsweise die Dosierung des abgegebenen Insulins. Durch die mit dem erfindungsgemäßen Biosensor 2 mögliche kontinuierliche Glukosemessung wird eine geschlossene Regelschleife für die Insulindosierung realisierbar.

Die Glukosemesswerte können im implantierten Therapiegerät (IMD) gespeichert oder mittels einer geeigneten Kommunikationsverbindung an das externe Patientengerät 4 und von diesem an das Home-Monitoring-System 8 übertragen werden. Aus den Glukosemesswerten können ganz oder teilweise im implantierten Therapiegerät (IMD), im Patientengerät 4 oder im Home-Monitoring-System 8 Parameter bestimmt oder Trends und Trendparameter ermittelt werden. Auch können die Glukosekonzentrationen oder die Trendparameter ganz oder teilweise im implantierten Therapiegerät (IMD), im Patientengerät 4 oder im Home-Monitoring-System 8 mit geeigneten Schwellwerten verglichen und Alarmmeldungen generiert werden. Die Glukosewerte und Trends können auch zum Monitoring des Krankheitsverlaufs oder der Therapie durch den Patienten oder den behandelnden Arzt verwendet werden, beispielsweise die Einhaltung einer bestimmten Diät.

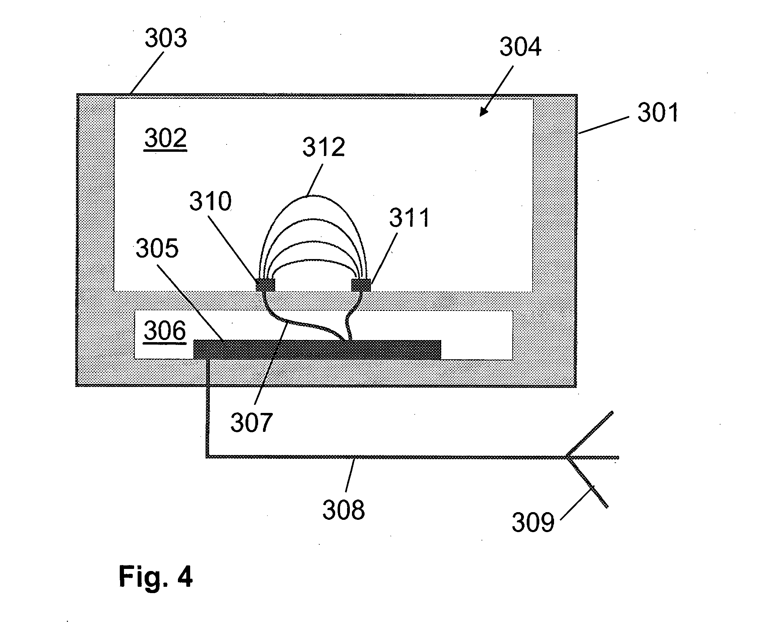

Es wird nun Bezug auf Figur 4 genommen, worin anhand einer schematischen Schnittdarstellung ein dritter Biosensor 2 veranschaulicht ist.

Der Biosensor 2 ist ein implantierbarer, drahtlos auslesbarer, passiver Sensor, der vorrangig zur Bestimmung des Glukosegehalts im Blut oder in der Bindegewebsflüssigkeit eingesetzt werden kann. Der Biosensor 2 wird an geeigneter Stelle, beispielsweise in einem Blutgefäß, implantiert. Er umfasst eine Kapsel 301, die aus einem geeigneten biokompatiblen Material gefertigt ist und eine Messkammer bzw. erste Kammer 302 umgibt. Auf einer Seite ist die Kapsel 301 bzw. erste Kammer 302 von einer geeigneten halbdurchlässigen Membran 303 abgeschlossen. Die erste Kammer 302 der Kapsel 301 ist mit einer geeigneten Testflüssigkeit 304 gefüllt, beispielsweise mit einer Mischung aus einem geeigneten Polymer und Concanavalin A als glukosebindendes Molekül, wobei die Testflüssigkeit 304 die Eigenschaft besitzt, ihre elektrischen Eigenschaften wie Leitfähigkeit oder Permittivität selektiv in Abhängigkeit vom pH-Wert zu verändern. Die Membran 303 ist so beschaffen, dass sie den Durchtritt von Wasserstoff-Ionen ermöglicht und gleichzeitig den Austritt der Testflüssigkeit 304 verhindert, so dass sich ein Gleichgewicht der Wasserstoffionen zwischen der Testflüssigkeit 304 in der Kapsel 301 und der umgebenden Körperflüssigkeit ausbildet. Damit kann über eine Leitfähigkeitsmessung in der ersten Kammer 302 der pH-Wert im Blut bestimmt werden. Die Messung der Leitfähigkeit erfolgt mittels eines mikroakustischen Sensors, bspw. eines SAW-Sensors 305. Zu diesem Zweck befinden sich in der ersten Kammer 302 geeignet angebrachte erste und zweite Elektroden 310, 311, die beispielsweise als Interdigitalelektroden ausgeführt sein können, so dass das elektrische Streufeld 312 zwischen diesen Elektroden 310, 311 ausreichend weit in die Testflüssigkeit 304 hineinreicht. Die beiden Elektroden 310, 311 sind über entsprechende erste Kabel 307 mit den Anschlüssen eines geeigneten Reflektors auf dem SAW-Sensor 305 verbunden. Die Wechselspannungs-Impedanz zwischen den beiden Elektroden 310, 311 beeinflusst mit ihrem Real- und Imaginärteil ($Z=R+jX$) die Reflexionseigenschaften des mit ihnen verbundenen Reflektors auf dem SAW-Sensor 305 hinsichtlich Betrag und Phase.

Der SAW-Sensor 305 enthält darüber hinaus mindestens einen weiteren Reflektor, mit dessen Hilfe eine Temperaturmessung und Temperaturkorrektur ermöglicht wird. Mit der erfassten Temperatur kann die Temperaturabhängigkeit des SAW-Sensors 305 und die der elektrischen Eigenschaften der Testflüssigkeit 304 kompensiert werden. Der SAW-Sensor 305 ist mit einem zweiten Kabel 308 mit einer oder mehreren geeigneten Sensorantennen 309 verbunden. Über diese Sensorantenne(n) 309 wird der Sendeimpuls 5 der Abfrageeinheit 3 eingekoppelt und die Echos 6 des SAW-Sensors 305 wieder abgestrahlt, um von der Auswerte- bzw. Abfrageeinheit 3 empfangen zu werden.

Die Auswerte- bzw. Abfrageeinheit 3, die hier beispielsweise Bestandteil des externen Patientengeräts 4 ist, sendet einen oder mehrere Sendeimpulse 5 aus und empfängt die Echos 6 vom implantierten Biosensor 2. Mindestens ein Echo 6 enthält die Information über die Leitfähigkeit der Testflüssigkeit 304 und mindestens ein Echo 6 enthält die Temperaturinformation. Die Abfrageeinheit 3 ermittelt diese Informationen aus den Eigenschaften der Echos 6, beispielsweise über eine Bestimmung der Amplituden und der Phasenlagen der Echos 6, erzeugt jeweils einen Messwert für die Leitfähigkeit und einen für die Temperatur und führt eine Temperaturkorrektur des Leitfähigkeitsmesswertes durch. Aus dem temperaturkorrigierten Leitfähigkeitsmesswert wird dann mittels des Zusammenhangs zwischen der Leitfähigkeit der Testflüssigkeit 304 und dem pH-Wert der pH-Wert im Blut ermittelt. Diese Abfrage kann zyklisch, beispielsweise alle 5 Minuten, in bestimmten Intervallen oder zu bestimmten Zeitabschnitten oder in Abhängigkeit von der körperlichen Aktivität des Patienten erfolgen. Das Patientengerät 4 kann anhand der Messwerte für den pH-Wert notwendige Therapiemaßnahmen einleiten, den gemessenen pH-Wert auf dem Display 9 anzeigen oder die pH-Werte abspeichern und über die Kommunikationsverbindung 7 an das Home-Monitoring-System 8 übertragen. Aus den pH-Werten können Trends und Trendparameter im Therapie- bzw. Patientengerät 4 oder im Home-Monitoring-System 8 ermittelt werden, die wiederum auf dem Display 9 des Therapie- bzw. Patientengeräts 4 angezeigt werden können. Auch können die pH-Werte oder die Trendparameter im Therapie- bzw. Patientengerät 4 oder im Home-Monitoring-System 8 mit geeigneten Schwellwerten verglichen und Alarmmeldungen generiert werden. Die pH-Werte und Trends können auch zum Monitoring des Krankheitsverlaufs oder der Therapie durch den Patienten oder den behandelnden Arzt verwendet werden.

In der Erfindung sind implantierbare, drahtlos auslesbare, batterielose Biosensoren zum Messen der Konzentration eines Analyten (beispielsweise Glukose, Biomarker, Hormone, Enzyme oder Proteine) oder einer Ionensorte (beispielsweise pH-Wert oder Kalziumionen) in einer wässrigen Lösung (beispielsweise im Blut oder in der Bindegewebsflüssigkeit) beschrieben. Die erfindungsgemäßen Biosensoren arbeiten auf der Basis von Hydrogelen bekannter Art, und nutzen deren bekannte Änderungen ihrer physikalischen Parameter, wie beispielsweise Volumenänderungen, Viskositätsänderungen oder Änderungen der elektrischen und dielektrischen Eigenschaften, in Abhängigkeit der Konzentration des Analyten oder Ionensorte. Die Biosensoren bestehen aus einer geeignet gestalteten Messkammer, die eine für die Konzentrationsmessung des Analyten bzw. Ionensorte geeignetes Hydrogel, enthält. Diese Messkammer ist an mindestens einer Fläche von einer geeigneten semipermeablen Membran abgeschlossen, die den Durchtritt des Analyten bzw. Ionensorte in die Messkammer ermöglicht und den Austritt der Testflüssigkeit bzw. der Messlösung zuverlässig verhindert. Dadurch baut sich zwischen der Umgebung und dem Innenraum der Messkammer ein Konzentrationsgleichgewicht des Analyten auf.

Im Unterschied zu den bekannten Vorrichtungen werden die Änderungen der physikalischen Parameter der Testflüssigkeit bzw. Hydrogele mit rein passiv arbeitenden SAW-Sensoren, die drahtlos abgefragt werden können, gemessen. Das bietet den Vorteil, dass die erfindungsgemäßen Biosensoren keine Messelektronik und keine Vorrichtungen für eine aktive Telemetrie benötigen, wodurch keine Batterien oder sonstige Energiespeicher erforderlich sind.

Die in dem erfindungsgemäßen Biosensor eingesetzten SAW-Sensoren können als Verzögerungsleitung oder als One-Port-Resonator ausgebildet sein. Der geeignete Bereich für die Resonanzfrequenz der SAW-Sensoren beträgt ca. 30 MHz bis ca. 10 GHz, wobei Frequenzen im ISM-Band (433 MHz) oder im MICS-Band (402-405 MHz) für implantierbare Sensoren bevorzugt werden. Da die Eigenschaften der SAW-Sensoren auch von der Temperatur beeinflusst werden, werden in den erfindungsgemäßen Biosensoren einer oder mehrere SAW-Sensoren verwendet, wobei mindestens einer keinen wirksamen Kontakt zum Messmedium aufweist und in geeigneter Weise zur Temperaturkompensation oder Temperaturmessung herangezogen wird. Auch kann mindestens ein SAW-Sensor mit mindestens zwei Reflektoren verwendet werden, wobei mindestens einer der Reflektoren zur Erzeugung von Signalen zur Temperaturmessung oder Temperaturkompensation verwendet wird.

Für Biosensoren, die eine Volumenänderung der Testflüssigkeit, beispielsweise Hydrogel, oder eine Änderung des osmotischen Drucks in Abhängigkeit der Konzentration des Ana-lyten oder Ionensorte ausnutzen, können die zur Druckmessung verwendeten SAW-Sensoren so angeordnet sein, dass mindestens einer den Druck in der Messkammer bestimmt während mindestens ein anderer den Umgebungsdruck außerhalb der Messkammer bestimmt. Die Differenz der beiden Drücke entspricht dann dem durch das Quellen der Testflüssigkeit, beispielsweise Hydrogels, oder durch Osmose aufgebauten Drucks in der Messkammer, aus dem die Konzentration des Analyten oder Ionensorte bestimmt wird. Die Verwendung dieser beiden Messwerte ermöglicht darüber hinaus eine Temperaturkompensation.

Bei Biosensoren, die die Viskositätsänderung der Hydrogele in Abhängigkeit von der Konzentration des Analyten oder Ionensorte ausnutzen, kann die Temperaturmessung mit dem dafür verwendeten SAW-Sensor auch benutzt werden, um neben der Temperaturabhängigkeit der SAW-Sensoren auch die Temperaturabhängigkeit der Viskosität der Testflüssigkeit, beispielsweise Hydrogels, oder der Messlösung zu kompensieren.

Bezugszeichenliste

| | |
|---|---|
| 1 | Sensoranordnung |
| 2 | Biosensor |
| 3 | Abfrageeinheit |
| 4 | Patientengerät |
| 5 | Sendeimpuls |
| 6 | Echo |
| 7 | Kommunikationsverbindung |
| 8 | Home-Monitoring-System |
| 9 | Display |
| 10 | Geräteantenne |
| 101 | Kapsel |
| 102 | erste Kammer |
| 103 | Membran |
| 104 | Testflüssigkeit |
| 105 | erster SAW-Sensor |
| 106 | zweite Kammer |
| 107 | zweiter SAW-Sensor |
| 108 | Kabel |
| 109 | Sensorantenne |
| 201 | Kapsel |
| 202 | erste Kammer |
| 203 | Membran |
| 204 | Testflüssigkeit |
| 205 | erster SAW-Sensor |
| 206 | zweite Kammer |
| 207 | zweiter SAW-Sensor |
| 208 | Kabel |
| 209 | Sensorantenne |
| 210 | erste Druckmessmembran |
| 211 | zweite Druckmessmembran |
| 212 | erster Stößel |
| 213 | zweiter Stößel |
| 301 | Kapsel |
| 302 | erste Kammer |
| 303 | Membran |
| 304 | Testflüssigkeit |
| 305 | SAW-Sensor |
| 306 | zweite Kammer |
| 307 | erste Kabel |
| 308 | zweite Kabel |
| 309 | Sensorantenne |
| 310 | erste Elektrode |
| 311 | zweite Elektrode |
| 312 | elektrisches Streufeld |

**Patentansprüche**

1. Implantierbarer Biosensor (2), welcher eine mit einem Hydrogel (204) gefüllte Messkammer (202) umfasst, wobei das Hydrogel (204) in der Lage ist, eine Änderung der Konzentration eines vorbestimmten Analyten oder Ionensorte in eine Druckänderung umzuwandeln, und wobei die Messkammer ( 202) durch wenigstens eine Membran (203) abgeschlossen ist, die für den Analyten oder Ionensorte durchlässig und für das Hydrogel (204) undurchlässig ist, wobei der Biosensor (2) mit wenigstens einem mikroakustischen Sensor (205) ausgerüstet ist, der mit dem Hydrogel (204) so wirkverbunden Ist, dass er die mit der Konzentration des Analyten oder Ionensorte sich ändernde Druckänderung detektieren kann, wobei er eine mit einem Hydrogel (204) gefüllte erste Kammer (202) und eine mit dem Hydrogel (204) nicht gefüllte zweite Kammer (206) aufweist, und wobei die beiden Kammern (202, 206) wenigstens abschnittsweise durch eine bei einem Druckunterschied in den beiden Kammern (202, 206) sich verformende erste Druckmessmembran (210) voneinander getrennt sind, **dadurch gekennzeichnet, dass** die zweite Kammer (206) wenigstens abschnittsweise durch eine bei einem Druckunterschied der zweiten Kammer (206) gegenüber Umgebung sich verformende zweite Druckmessmembran (211) von der Umgebung getrennt ist, und wobei der mikroakustische Sensor (205) in der zweiten Kammer (206) aufgenommen und so mit den beiden Druckmessmembranen (210, 211) wirkverbunden ist, dass er deren Verformung detektieren kann.

2. Implantierbarer Biosensor (2) nach Anspruch 1, **dadurch gekennzeichnet, dass** der mikroakustische Sensor (205) als ein mit der wirkverbundenen Druckmessmembran (210, 211) mechanisch gekoppeltes biegbares Organ ausgebildet ist.

3. Implantierbarer Biosensor (2) nach einem der Ansprüche 1 bis 2, **dadurch gekennzeichnet, dass** ein zweiter mikroakustischer Sensor in der zweiten Kammer (206) aufgenommen ist, der die Temperatur detektieren kann.

4. Implantierbarer Biosensor (2) nach einem der Ansprüche 1 bis 3, **dadurch gekennzeichnet, dass** das Hydrogel (204) in der Lage ist, eine Änderung der Konzentration eines Analyten, insbesondere Glucose, oder Ionensorte in eine Änderung seines Volumens umzuwandeln.

5. Sensoranordnung (1) mit wenigstens einem implantierbarer Biosensor (2) nach einem der Ansprüche 1 bis 4, **gekennzeichnet durch** eine mit dem Biosensor (2) drahtlos gekoppelte Abfrageeinrichtung (3).

**6.** Sensoranordnung (1) nach Anspruch 5, **dadurch gekennzeichnet, dass** die Abfrageeinrichtung (3) in ein Therapiegerät (4) integriert ist.

**7.** Sensoranordnung (1) nach Anspruch 6, **dadurch gekennzeichnet, dass** das Therapiegerät (4) mit einem Home-Monitoring-System (8) gekoppelt ist.

**Claims**

**1.** An implantable biosensor (2), which comprises a measurement chamber (202) filled with a hydrogel (204), wherein the hydrogel (204) is able to convert a change in the concentration of a predetermined analyte or ion type into a pressure change, and wherein the measurement chamber (202) is closed by at least one membrane (203), which is permeable for the analyte or ion type and is impermeable for the hydrogel (204), wherein the biosensor (2) is equipped with at least one microacoustic sensor (205), which is operatively connected to the hydrogel (204) such that it can detect changing pressure changes using the concentration of the analyte, wherein it has a first chamber (202) filled with a hydrogel (204) and a second chamber (206) not filled with the hydrogel (204), and wherein the two chambers (202, 206) are separated from one another at least in portions by a first pressure measurement membrane (210) that deforms in the case of a pressure difference between the two chambers (202, 206), **characterised in that** the second chamber (206) is separated from the surrounding environment at least in portions by a second pressure measurement membrane (211) that deforms in the case of a pressure difference between the second chamber (206) and the surrounding environment, and wherein the microacoustic sensor (205) is accommodated in the second chamber (206) and is operatively connected to the two pressure measurement membranes (210, 211) such that it can detect deformation thereof.

**2.** The implantable biosensor (2) according to Claim 1, **characterised in that** the microacoustic sensor (205) is formed as a flexible member coupled mechanically to the operatively connected pressure measurement membrane (210, 211).

**3.** The implantable biosensor (2) according to one of Claims 1 to 2, **characterised in that** a second microacoustic sensor is accommodated in the second chamber (206) and can detect the temperature.

**4.** The implantable biosensor (2) according to one of Claims 1 to 3, **characterised in that** the hydrogel (204) is able to convert a change in the concentration of an analyte, in particular glucose, or ion type into a change in the volume thereof.

**5.** A sensor arrangement (1) having at least one implantable biosensor (2) according to one of Claims 1 to 4, **characterised by** an interrogator (3) coupled wirelessly to the biosensor (2).

**6.** The sensor arrangement (1) according to Claim 5, **characterised in that** the interrogator (3) is integrated in a therapy device (4).

**7.** The sensor arrangement (1) according to Claim 6, **characterised in that** the therapy device (4) is coupled to a home monitoring system.

**Revendications**

**1.** Biocapteur implantable (2) comprenant une chambre de mesure (202) remplie d'hydrogel (204), dans lequel l'hydrogel (204) est capable de convertir une modification de la concentration d'un analyte prédéterminé ou d'une sorte d'ion en une modification de pression, et dans lequel la chambre de mesure (202) est fermée par au moins une membrane (203), laquelle est perméable à l'analyte ou à la sorte d'ion et imperméable à l'hydrogel (204), dans lequel le biocapteur (2) est équipé d'au moins un capteur microacoustique (205) fonctionnellement relié à l'hydrogel (204), de manière à pouvoir détecter la modification de pression variant avec la concentration de l'analyte ou de la sorte d'ion, dans lequel il comporte une première chambre (202) remplie avec l'hydrogel (204) et une deuxième chambre (206) non remplie avec l'hydrogel (204), et dans lequel les deux chambres (202, 206) sont séparées l'une de l'autre au moins par sections, par une première membrane de mesure de pression (210) qui se déforme lors d'une différence de pression dans les deux chambres (202, 206), **caractérisé en ce que** la deuxième chambre (206) est séparée de l'environnement au moins par sections par une deuxième membrane de mesure de pression (211) qui se déforme lors d'une différence de pression entre la deuxième chambre (206) et l'environnement, et dans lequel le capteur microacoustique (205) est accueilli dans la deuxième chambre (206) et relié fonctionnellement aux deux membranes de mesure de pression (210, 211) de manière à pouvoir détecter leur déformation.

**2.** Biocapteur implantable (2) selon la revendication 1, **caractérisé en ce que** le capteur microacoustique (205) est conçu comme un organe flexible accouplé mécaniquement à la membrane de mesure de pression (210, 211) reliée fonctionnellement.

**3.** Biocapteur implantable (2) selon l'une des revendications 1 à 2, **caractérisé en ce qu'**un deuxième capteur microacoustique capable de détecter la température est accueilli dans la deuxième chambre (206).

4. Biocapteur implantable (2) selon l'une des revendications 1 à 3, **caractérisé en ce que** l'hydrogel (204) est capable de convertir une modification de la concentration d'un analyte, en particulier du glucose, ou d'une sorte d'ion, en une modification de son volume.

5. Système de capteur (1) avec au moins un biocapteur implantable (2) selon l'une des revendications 1 à 4, **caractérisé par** un dispositif d'interrogation (3) accouplé sans fil au biocapteur (2).

6. Système de capteur (1) selon la revendication 5, **caractérisé en ce que** le dispositif d'interrogation (3) est intégré dans un appareil thérapeutique (4).

7. Système de capteur (1) selon la revendication 6, **caractérisé en ce que** l'appareil thérapeutique (4) est accouplé à un système de Home Monitoring.

Fig. 1

Fig. 2

**Fig. 3**

EP 2 158 840 B1

**Fig. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6475750 B1